# EUROPÄISCHE PATENTSCHRIFT

(11) **EP 1 192 931 B1**
(45) Veröffentlichungstag und Bekanntmachung des Hinweises auf die Patenterteilung: **03.09.2003**
(21) Anmeldenummer: 01128662.2
(22) Anmeldetag: 01.12.2001
(51) Int. Cl.: A61K 7/06, A61K 7/11

(54) **Haarbehandlungsmittel mit einer Kombination von drei Polymeren**
Hair treatment composition comprising a combination of three polymers
Composition de traitement capillaire comprenant une combinaison de trois polymers

(43) Veröffentlichungstag der Anmeldung: 03.04.2002
(73) Patentinhaber: Wella Aktiengesellschaft, 64295 Darmstadt (DE)
(72) Erfinder: Gänger, Klaus, 64319 Pfungstadt (DE); Florig, Ellen, 64689 Grasellenbach (DE)

(56) Entgegenhaltungen:
- WO-A-00/06092
- WO-A-98/19653
- DE-A- 3 044 754
- DE-A- 4 034 315
- DE-A- 4 316 242
- US-A- 5 985 295
- PATENT ABSTRACTS OF JAPAN vol. 018, no. 482 (C-1247), 8. September 1994 (1994-09-08) & JP 06 157249 A (LION CORP), 3. Juni 1994 (1994-06-03)

## Beschreibung

Gegenstand der Erfindung ist ein Haarbehandlungsmittel mit einem Gehalt an einer Kombination von mindestens drei filmbildenden und haarfestigenden Polymeren und zwar mit mindestens einem amphoteren Polymer, mindestens einem anionischen Polymer und mindestens einem nichtionischen Vinyllactam Homo- oder Copolymer.

Um dem menschlichen Haar Festigung und Halt zu geben oder um eine erstellte Frisur zu stabilisieren, werden Haarbehandlungsmittel in Form von Festigerlotionen, Aerosol- und Non-Aerosolsprays, Festigerschäumen, Gelen etc. eingesetzt. Die für diese Zwecke üblicherweise verwendeten kosmetischen, haarfestigenden Polymere zeigen in wässrigen, alkoholischen oder wässrig-alkoholischen Medien gute Festigungseigenschaften, die nach der Anwendung die Haare in Form halten, festigen und die erstellte Frisur stabilisieren. Häufig ist damit aber ein starrer, unnatürlicher Griff des Haares verbunden, die Elastizität der Polymerfilme oder der polymervernetzten Frisur ist ungenügend oder die Dauerhaftigkeit der Halt- und Festigungsleistungen ist nicht zufriedenstellend. Es ist bekannt, zwei Polymere zu kombinieren, um die Eigenschaften von Polymerfilmen zu modifizieren. Aus der EP 0 507 896 A ist ein Mittel zur Haarfestigung bekannt mit einem Gehalt an einer Kombination eines amphoteren mit einem nichtionischen Polymer. Die Kombination hat gute Festigungseigenschaften bei reduzierter Klebrigkeit und guter Auswaschbarkeit.

Es bestand die Aufgabe, die filmbildenden und haarfestigenden Eigenschaften von polymerhaltigen Zubereitungen weiter zu verbessern und insbesondere die Elastizität von Polymerfilmen bzw. von polymerbehandelten Haaren zu steigern und die Dauerhaftigkeit der positiven Eigenschaften zu verbessern.

Es wurde nun gefunden, dass die Aufgabe gelöst wird durch ein Haarbehandlungsmittel mit einer Kombination aus drei ausgewählten Polymeren. Gegenstand der Erfindung ist ein Haarbehandlungsmittel mit einem Gehalt an
(A) mindestens einem filmbildenden, haarfestigenden, amphoteren Copolymeren, welches gebildet ist aus mindestens einer ersten Monomerart, welche mindestens eine neutralisierte oder nicht neutralisierte Säuregruppe aufweist und mindestens einer zweiten Monomerart, welche mindestens eine neutralisierte oder nicht neutralisierte Amingruppe aufweist,
(B) mindestens einem zweiten filmbildenden, haarfestigenden anionischen Polymer und
(C) mindestens einem dritten, filmbildenden, haarfestigenden nichtionischen Polymer, ausgewählt aus Vinyllactam Homooder Copolymeren.
Unter filmbildenden und haarfestigenden Polymeren werden erfindungsgemäß solche Polymere verstanden, die bei Anwendung in 0,01 bis 5%iger wässriger, alkoholischer oder wässrig-alkoholischer Lösung in der Lage sind, auf dem Haar einen Polymerfilm abzuscheiden und auf diese Weise das Haar zu festigen bzw. die Wellstabilität zu erhöhen. Die erfindungsgemäße Polymerkombination bewirkt eine gute Haarfestigung bei guten Griffeigenschaften, guter Elastizität und verbesserter Dauerhaftigkeit der Wirkungen.

Das amphotere Polymer (A) ist in dem erfindungsgemäßen Mittel vorzugsweise in einer Menge von 1 bis 15, besonders bevorzugt von 2 bis 10 Gew.%, das anionische Polymer (B) vorzugsweise in einer Menge von 0,01 bis 5, besonders bevorzugt von 0,02 bis 2 Gew.% und das nichtionische Polymer (C) vorzugsweise in einer Menge von 0,01 bis 5, besonders bevorzugt von 0,02 bis 2 Gew.% enthalten. Die Mengenverhältnisse der drei Polymere zueinander sind so gewählt, dass das Mengenverhältnis von amphoterem Polymer (A) zum anionischen Polymer (B) vorzugsweise von 1:1 bis 200:1, besonders bevorzugt von 20:1 bis 150:1, das Mengenverhältnis von amphoterem Polymer (A) zum nichtionischen Polymer (C) vorzugsweise von 1:1 bis 200:1, besonders bevorzugt von 20:1 bis 150:1 und das Mengenverhältnis von anionischem Polymer (B) zum nichtionischen Polymer (C) vorzugsweise von 0,5:1 bis 2:1, besonders bevorzugt von 0,7:1 bis 1,5:1 beträgt.

Geeignete amphotere Polymere (A) können Homo- oder Copolymere sein, welche sowohl kationisierbare Amingruppen als auch anionische oder anionisierbare Säuregruppen enthalten, wobei die kationischen bzw. kationisierbaren Gruppen entweder in der Polymerkette oder vorzugsweise als Substituent an einem oder mehreren der Monomeren enthalten sind. Das amphotere Polymer kann mit neutralen Comonomeren copolymerisiert sein, die weder kationische bzw. kationisierbare Gruppen noch anionische bzw. anionisierbare Gruppen enthalten. Derartige neutrale Comonomere sind z.B. Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylcaprolactam, Vinylester, Vinylalkohol, Propylenglykol oder Ethylenglykol, wobei die Alkylgruppen dieser Monomere vorzugsweise C1 bis C7-Alkylgruppen, besonders bevorzugt C1- bis C3-Alkylgruppen sind.

Das amphotere Polymer kann ein Copolymer sein, welches gebildet ist aus mindestens einer ersten Monomerart, welche mindestens eine neutralisierte oder nicht neutralisierte Säuregruppe aufweist und mindestens einer zweiten Monomerart, welche mindestens eine neutralisierte oder nicht neutralisierte basische Gruppe aufweist. Geeignete Monomere des amphoteren Polymers, welche Säuregruppen aufweisen, sind ungesättigte, radikalisch polymerisierbare Verbindungen, welche mindestens eine Säuregruppe, z.B. eine Carbonsäuregruppe tragen, insbesondere Carboxyvinylmonomere wie z.B. Acrylsäure, Methacrylsäure, Crotonsäure oder Maleinsäure bzw. deren Monoester, von denen Acrylsäure und Methacrylsäure bevorzugt sind. Geeignete Monomere des amphoteren Polymers, welche neutralisierte oder nicht neutralisierte basische Gruppen aufweisen, sind ungesättigte, radikalisch polymerisierbare Verbindungen. Als basische Gruppen kommen insbesondere primäre, sekundäre oder tertiäre Amine in Betracht, wobei das N-Atom auch Teil eines Ringes sein kann. Beispiele für derartige amphotere Copolymere sind Copolymere gebildet aus Alkylacrylamid, Alkylaminoalkylmethacrylat und ein, zwei oder mehr Monomeren bestehend aus Acrylsäure, Methacrylsäure und deren einfachen Alkylestern, wobei mindestens eines der Monomere eine Säuregruppe trägt. Ein bevorzugtes amphoteres Polymer ist ein Copolymer aus Octylacrylamid, N-tert.-Butylaminoethylmethacrylat und zwei oder mehr Monomeren ausgewählt aus Acrylsäure, Methacrylsäure und deren Alkylestern, wobei mindestens eines der Monomere eine Säuregruppe trägt. Entsprechende Handelsprodukte sind Amphomer® oder Amphomer® LV-71.

Geeignete anionische Polymere (B) sind solche Polymere, welche Säuregruppen wie z.B. Carbonsäure-, Sulfonsäure- oder Phosphorsäuregruppen enthalten, welche mittels üblicher Basen wie z.B. organischer Amine oder Alkali- oder Erdalkalihydroxide teilweise oder vollstänidg deprotoniert sind. Die Säuregruppen sind vorzugsweise zu 50 bis 100 %, besonders bevorzugt zu 70 bis 100% neutralisiert.

Das anionische Polymer kann ein Homo- oder Copolymer mit Säuregruppen enthaltenden Monomereinheiten auf natürlicher oder synthetischer Basis sein, welches gegebenenfalls mit Comonomeren, die keine Säuregruppen enthalten, copolymerisiert ist. Als Säuregruppen sind die Carbonsäuregruppen bevorzugt. Geeignete Säuregruppen enthaltende Monomere sind beispielsweise Acrylsäure, Methacrylsäure, Crotonsäure, Maleinsäure bzw. Maleinsäureanhydrid, welches nach Polymerisation hydrolysiert oder teilverestert wird, Maleinsäuremonoester, insbesondere die Mono-C1-C7-alkylester der Maleinsäure sowie Aldehydocarbonsäuren oder Ketocarbonsäuren.

Nicht mit Säuregruppen substituierte Comonomere sind z.B. Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylcaprolactam, Vinylester, Vinylalkohol, Propylenglykol oder Ethylenglykol, aminsubstituierte Vinylmonomere wie z.B. Dialkylaminoalkylacrylat oder -acrylamid, Dialkylaminoalkylmethacrylat oder -methacrylamid, Monoalkylaminoalkylacrylat oder -acrylamid und Monoalkylaminoalkylmethacrylat oder -methacrylamid, wobei die Alkylgruppen dieser Monomere vorzugsweise C1- bis C7-Alkylgruppen, besonders bevorzugt C1- bis C3-Alkylgruppen sind.

Geeignete Polymere mit Säuregruppen sind insbesondere unvernetzte oder mit polyfunktionellen Agenzien vernetzte Homopolymere der Acrylsäure oder der Methacrylsäure, Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte Polymere mit Säuregruppen sind vernetzte oder unvernetzte Vinylacetat/Crotonsäure Copolymere (INCI-Bezeichnung: VA/Crotonates Copolymer), Vinylacetat/Crotonsäure/Vinylalkanoat Copolymere (INCI-Bezeichnungen: VA/Crotonates/Vinyl Propionate Copolymer, VA/Crotonates/Vinyl Neodecanoate Copolymer), Copolymere aus ein oder mehreren C1-C5-Alkylacrylaten, insbesondere C2-C4-Alkylacrylaten und Acrylsäure oder Methacrylsäure (INCI-Bezeichnung: Acrylates Copolymer), Terpolymere aus Acrylsäure, Alkylacrylat und N-Alkylacrylamid, insbesondere Acrylsäure/Ethylacrylat/N-t-Butylacrylamid Terpolymere (INCI-Bezeichnung: Acrylates/Acrylamide Copolymer), Copolymere aus Methylvinylether und Maleinsäuremonoalkylestern (INCI-Bezeichnungen: Ethylester of PVM/MA Copolymer, Butylester of PVM/MA Copolymer).

Die sauren Gruppen der Polymere der Komponenten (A) und (B) können teilweise oder vollständig mit einem basischen Neutralisationsmittel neutralisiert sein. Der bevorzugte Neutralisationsgrad ist 50 bis 100 %, besonders bevorzugt 70-100%. Als Neutralisationsmittel können organische oder anorganische Basen verwendet werden. Geeignete Neutralisationsmittel sind insbesondere Alkalihydroxide wie Natrium- oder Kaliumhydroxid oder Aminoalkohole wie 2-Amino-2-methyl-1-propanol (AMP), Triethanolamin, Triisopropanolamin, Monoethanolamin; Diethanolamin, Tri-(2-hydroxy-1-propyl)amin, 2-Amino-2-methyl-2-propan-1,3-diol oder 2-Amino-2-hydroxymethyl-propan-1,3-diol.

Bei den nichtionischen Polymeren (C) handelt es sich um Homooder Copolymere, die vollständig oder teilweise aus Vinyllactammonomeren aufgebaut sind. Bevorzugte Vinyllactame sind N-Vinylpyrrolidon und N-Vinylcaprolactam. Als Comonomere kommen Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol infrage, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams oder des Vinylpyrrolidons. Weitere geeignete synthetische filmbildende, nichtionische, haarfestigende Polymere sind z.B. Copolymerisate aus Vinylpyrrolidon und Vinylacetat sowie Terpolymere aus Vinylpyrrolidon, Vinylacetat und Vinylpropionat. Besonders bevorzugt sind Polyvinylpyrrolidon/Vinylacetat Copolymere.

Das erfindungsgemäße Mittel kann in einem alkoholischen, wässrig-alkoholischen oder in einem wässrigen Medium konfektioniert sein. Bevorzugt ist ein alkoholisches Lösungsmittel, wobei als Alkohole insbesondere die für kosmetische Zwecke üblicherweise verwendeten niederen Alkohole mit 1 bis 4 C-Atomen wie z.B. Ethanol und Isopropanol enthalten sind. Diese Alkohole können in einer Menge von 15 bis 85 Gew.%, vorzugsweise von 25 bis 75 Gew.% enthalten sein. Als zusätzliche Co-Solventien können organische Lösungsmittel oder ein Gemisch aus Lösungsmitteln mit einem Siedepunkt unter 400°C in einer Menge von 0,1 bis 15 Gew.%, bevorzugt von 1 bis 10 Gew.% enthalten sein. Besonders geeignet als zusätzliche Co-Solventien sind unverzweigte oder verzweigte Kohlenwasserstoffe wie Pentan, Hexan, Isopentan und cyclische Kohlenwasserstoffe wie Cyclopentan und Cyclohexan. Mögliche wasserlösliche Lösungsmittel sind Glycerin, Ethylenglykol und Propylenglykol in einer Menge bis 30 Gew.%.

Das erfindungsgemäße Mittel kann darüber hinaus die für Haarbehandlungsmittel üblichen Zusatzbestandteile enthalten, z.B. Netzmittel, Emulgatoren oder Lösungsvermittler aus den Klassen der nichtionischen, anionischen, kationischen oder amphoteren oberflächenaktiven Tenside in einer Menge von 0,1 bis 15 Gew.%; Feuchthaltemittel; Parfümöle in einer Menge von 0,1 bis 0,5 Gew.%; Pflanzen- und Kräuterextrakte in einer Menge von 0,1 bis 5 Gew.%; bakterizide und fungizide Wirkstoffe in einer Menge von 0,01 bis 1,0 Gew.%; Weichmacher wie z.B. Phtalsäureester, oder Alkylcitrate, Silikonverbindungen, wie z.B. flüchtige oder nicht-flüchtige Silikonöle, hochmolekulare Siloxanpolymere oder Copolymere von Siloxanen und Ethylen und/oder Propylenglykol in einer Menge von 0,05 bis 20 Gew.%; Lichtschutzmittel, Antioxidantien, Radikalfänger, Antischuppenwirkstoffe, in einer Menge von etwa 0,01 bis 2 Gew.%; Glanzgeber, Vitamine, Kämmbarkeitsverbesserer, rückfettende Agenzien und Entschäumer, soweit derartige Zusätze für die jeweilige Applikationsform zweckmäßig sind.

Das erfindungsgemäße Mittel kann in verschiedenen Applikationsformen Anwendung finden, z.B. als Lotion, Non-Aerosol Sprühlotion, welche mittels einer mechanischen Vorrichtung zum Versprühen zum Einsatz kommt, als Aerosol-Spray welches mittels eines Treibmittels versprüht wird, als treibmittelhaltige Aerosol-Schaumzusammensetzung oder als Non-Aerosol Schaumzusammensetzung, welche in Kombination mit einer geeigneten mechanischen Vorrichtung zum Verschäumen vorliegt, als Gel, als Flüssiggel, als versprühbares Gel oder als Schaumgel. Auch ein Einsatz in Form einer mit einem üblichen Verdicker verdickten Lotion ist möglich.

Eine bevorzugte Ausführungsform ist ein Aerosol Haarspray. Wenn das erfindungsgemäße Haarbehandlungsmittel in Form eines Aerosolsprays vorliegt, so enthält es zusätzlich 15 bis 85 Gew.%, bevorzugt 25 bis 75 Gew.% eines Treibmittels und wird in einem Druckbehälter abgefüllt. Als Treibmittel sind beispielsweise niedere Alkane, wie z.B. n-Butan, i-Butan und Propan, oder auch deren Gemische sowie Dimethylether oder Fluorkohlenwasserstoffe wie F 152a (1,1-Difluorethan) oder F 134 (Tetrafluorethan) sowie ferner bei den in Betracht kommenden Drücken gasförmig vorliegende Treibmittel, wie z.B. N₂, N₂O und CO₂ sowie Gemische der vorstehend genannten Treibmittel geeignet. Besonders bevorzugt sind Propan und Butan. Das Lösungsmittel ist vorzugsweise rein alkoholisch, kann aber auch bis zu 10 Gew.%, vorzugsweise 2 bis 8 Gew.% Wasser enthalten.

Ein besonders bevorzugtes Aerosol-Haarsprayprodukt besteht aus einer druckfesten Verpackung, einer Haarsprayzusammensetzung, welche zusammen mit einem Treibmittel in der druckfesten Verpackung abgefüllt ist und einem Sprühsystems zum Versprühen der Zusammensetzung, wobei die Zusamensetzung einen Gehalt aufweist an
(A) 1 bis 15 Gew.% eines zu 50 bis 100% neutralisierten amphoteren Copolymeren, gebildet aus Alkylacrylamid, Alkylaminoalkylmethacrylat und zwei oder mehr Monomeren bestehend aus Acrylsäure, Methacrylsäure und deren einfachen Alkylestern, wobei mindestens eines der Monomere eine Säuregruppe trägt und die Alkylgruppen 1 bis 10 C-Atome aufweisen,
(B) 0,01 bis 5 Gew.% eines zu 50 bis 100% neutralisierten anionischen Copolymeren, ausgewählt aus Polymeren, welche gebildet sind aus mindestens einer ersten Monomerart, ausgewählt aus Acryl- und Methacrylsäure und mindestens einer zweiten Monomerart, ausgewählt aus Acrylsäurealkylestern, Methacrylsäurealkylestern, Acrylamid und Methacrylamid, wobei die Alkylgruppen 1 bis 5 C-Atome aufweisen,
(C) 0,01 bis 5 Gew.% Vinylpyrrolidon/Vinylacetat Copolymer,
(D) 10 bis 60 Gew.% eines C2- oder C3-Alkohols und
(E) 10 bis 85 Gew.% mindestens eines Aerosol-Treibmittels, vorzugsweise Propan, Butan, Dimethylether oder einem Gemisch dieser Treibmittel.

Wenn das erfindungsgemäße Haarbehandlungsmittel in Form eines versprühbaren Non-Aerosol-Haarsprays vorliegt, so wird es mit Hilfe einer geeigneten mechanisch betriebenen Sprühvorrichtung versprüht. Unter mechanischen Sprühvorrichtungen sind solche Vorrichtungen zu verstehen, welche das Versprühen einer Zusammensetzung ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Wenn das erfindungsgemäße Haarbehandlungsmittel in Form einer Haarschaumzusammensetzung (Mousse) vorliegt, so enthält es mindestens eine übliche, hierfür bekannte schaumgebende Substanz. Das Mittel wird mit oder ohne Hilfe von Treibgasen oder chemischen Treibmitteln verschäumt und als Schaum in das Haar eingearbeitet und ohne Ausspülen im Haar belassen. Das erfindungsgemäße Mittel weist als zusätzliche Komponente eine Vorrichtung zum Verschäumen der Zusammensetzung auf. Unter Vorrichtungen zum Verschäuen sind solche Vorrichtungen zu verstehen, welche das Verschäumen einer Flüssigkeit mit oder ohne Verwendung eines Treibmittels ermöglichen. Als geeignete mechanische Schäumvorrichtung kann beispielsweise ein handelsüblicher Pumpschäumer oder ein Aerosolschaumkopf verwendet werden.

Wenn das erfindungsgemäße Haarbehandlungsmittel in Form eines Haargels vorliegt, so enthält es zusätzlich mindestens eine gelbildende Substanz in einer Menge von vorzugsweise 0,05 bis 10, besonders bevorzugt von 0,1 bis 2 Gew.%. Die Viskosität des Gels beträgt vorzugsweise von 500 bis 50.000 mPa s, besonders bevorzugt von 1.000 bis 15.000 mPa s bei 25°C (gemessen mit einem Rotationsviskosimeter RheoStress 100 der Firma Haake bei einer Temperatur von 25°C und einem Schergefälle 50 s⁻¹).

Wenn das erfindungsgemäße Haarbehandlungsmittel in Form einer Haarlotion vorliegt, so liegt es als im wesentlichen nicht-viskose oder gering viskose, fließfähige Lösung, Dispersion oder Emulsion mit einem Gehalt an mindestens 10 Gew.%, vorzugsweise 20 bis 95 Gew.% eines kosmetisch verträglichen Alkohols vor. Als Alkohole können insbesondere die für kosmetische Zwecke üblicherweise verwendeten niederen Alkohole mit 1 bis 4 C-Atomen wie z.B. Ethanol und Isopropanol verwendet werden.

Die nachfolgenden Beispiele sollen den Gegenstand der Erfindung näher erläutern. Die in den Beispielen angegebenen Polymergehalte beziehen sich jeweils auf den Feststoffgehalt.

### Beispiele

### Beispiel 1

| | A | B |
|---|---|---|
| Amphomer® ¹⁾ | 4,98 g | 4,98 g |
| Ultrahold® 8 ²⁾ | 0,05 g | - |
| PVP/VA Copolymer | 0,05 g | - |
| Aminomethylpropanol | 0,91 g | 0,91 g |
| PEG-12 Dimethicone | 0,59 g | 0,59 g |
| Parfüm | 0,15 g | 0,15 g |
| Triethylcitrat | 0,015 g | 0,015 g |
| Wasser | 2,45 g | 2,45 g |
| Ethanol | Ad 100 g | Ad 100 g |

| | | |
|---|---|---|
| ¹⁾ Octylacrylamid/Acrylsäure/Butylaminoethylmethacrylat/Methylmethacrylat/Hydroxypropylmethacrylat Copolymer | | |
| ²⁾ Ethylacrylat/Acrylsäure/N-tert.-Butylacrylamid Copolymer | | |

Mit der erfindungsgemäßen Zusammensetzung A, mit der Vergleichszusammensetzung B sowie mit einer unbehandelten Haarsträhne (Wasserwelle) wurden Wellstabilitätsmessungen (Curl Retention-Messungen) durchgeführt. Für die Messungen wurden je 3 einheitliche, standardisierte Zählhaarsträhnen pro Probe verwendet. Eine Zählhaarsträhne besteht aus 100 Einzelhaaren (Eurohaar, 16,5 cm lang, mittlerer Durchmesser 76,3 µm der Fa. Kerling). Die Messungen wurden an einfach gebleichten Strähnen nach zweimaliger Wäsche mit einem Standardshampoo (10% Natriumlaurylethersulfat, 4% NaCl) durchgeführt. Die bei 20°C und 65% relativer Luftfeuchtigkeit getrockneten Strähnen wurden mit 10 g Gewichten beschwert und auf Spiralwickler gewickelt, mit 100 µl der zu testenden Zusammensetzung behandelt und über Nacht bei 20°C und 85% relativer Luftfeuchtigkeit getrocknet. Die getrockneten Strähnen wurden von den Wicklern abgenommen, mit 50 mg Gewichten beschwert und bei 20°C / 85% relativer Luftfeuchtigkeit ausgehängt. Es wurde die Zeit gemessen, um eine Lockenaushängung von 10 mm zu erreichen.

| | | |
|---|---|---|
| Unbehandelt (Wasserwelle) | 2,8 Stunden | |
| Zusammensetzung B: | 5,6 Stunden | (+100%) |
| Zusammensetzung A: | 8,1 Stunden | (+189%) |

Die Standardzusammensetzung B führt gegenüber einer Wasserwelle zu einer doppelt so langen Haltbarkeit für 10 mm Aushängung. Die erfindungsgemäße Zusammensetzung B zeigt eine um weitere 89% verlängerte Haltbarkeit.

### Beispiel 2: Aerosolhaarspray

Die Zusammensetzung A wurde mit Propan/Butan-Treibmittel im Verhältnis 67:33 in einer Aerosolsprayverpackung abgefüllt.

### Beispiel 3: Aerosolhaarspray

| | |
|---|---|
| Amphomer® ¹⁾ | 4,98 g |
| Ultrahold® 8 ²⁾ | 0,13 g |
| PVP/VA Copolymer | 0,13 g |
| Aminomethylpropanol | 0,91 g |
| PEG-12 Dimethicone | 0,59 g |
| Parfüm | 0,15 g |
| Triethylcitrat | 0,02 g |
| Wasser | 2,45 g |
| Ethanol | Ad 100 g |

Die Zusammensetzung wurde mit Propan/Butan-Treibmittel im Verhältnis 67:33 in einer Aerosolsprayverpackung abgefüllt.

## Patentansprüche

1. Haarbehandlungsmittel mit einem Gehalt an
(A) mindestens einem filmbildenden, haarfestigenden, amphoteren Copolymeren, welches gebildet ist aus mindestens einer ersten Monomerart, welche mindestens eine neutralisierte oder nicht neutralisierte Säuregruppe aufweist und mindestens einer zweiten Monomerart, welche mindestens eine neutralisierte oder nicht neutralisierte Amingruppe aufweist,
(B) mindestens einem filmbildenden, haarfestigenden anionischen Polymer und
(C) mindestens einem filmbildenden, haarfestigenden nichtionischen Polymer, ausgewählt aus Vinyllactam Homo- oder Copolymeren.

2. Haarbehandlungsmittel nach Anspruch 1, **dadurch gekennzeichnet, dass** das amphotere Polymer (A) in einer Menge von 1 bis 15 Gew.%, das anionische Polymer (B) in einer Menge von 0,01 bis 5 Gew.% und das nichtionische Polymer (C) in einer Menge von 0,01 bis 5 Gew.% eingesetzt wird.

3. Haarbehandlungsmittel nach einem der vorhergehenden Ansprüche, **dadurch gekennzeichnet, dass** das Mengenverhältnis von amphoterem Polymer zum anionischen Polymer (A):(B) von 1:1 bis 200:1 und das Mengenverhältnis von amphoterem Polymer zum nichtionischen Polymer (A):(C) von 1:1 bis 200:1 beträgt.

4. Mittel nach einem der vorhergehenden Ansprüche, **dadurch gekennzeichnet, dass** das amphotere Polymer (A) ausgewählt ist aus Copolymeren, gebildet aus Alkylacrylamid, Alkylaminoalkylmethacrylat und ein, zwei oder mehr Monomeren bestehend aus Acrylsäure, Methacrylsäure und deren einfachen Alkylestern, wobei mindestens eines der Monomere eine Säuregruppe trägt, wobei die Alkylgruppen jeweils 1 bis 10 C-Atome aufweisen.

5. Mittel nach einem der vorstehenden Ansprüche, **dadurch gekennzeichnet, dass** das anionische Polymer (B) ausgewählt ist aus Polymeren oder Copolymeren, welche aufgebaut sind aus mindestens einer Monomerart, bei der es sich um eine radikalisch polymerisierbare, ethylenisch ungesättigte Carbonsäure handelt, welche ausgewählt ist aus Acrylsäure, Methacrylsäure, Crotonsäure, Maleinsäure, Maleinsäureanhydrid und Maleinsäuremonoestern.

6. Mittel nach Anspruch 5, **dadurch gekennzeichnet, dass** das anionische Polymer (B) ausgewählt ist aus Copolymeren, welche gebildet sind aus mindestens einer ersten Monomerart, ausgewählt aus Acryl- und Methacrylsäure und mindestens einer zweiten Monomerart, ausgewählt aus Acrylsäurealkylestern, Methacrylsäurealkylestern, Acrylamid, Methacrylamid und Estern von Vinylalkohol, wobei die Alkylgruppen 1 bis 5 C-Atome aufweisen.

7. Mittel nach einem der vorstehenden Ansprüche, **dadurch gekennzeichnet, dass** das nichtionische Polymer (C) ausgewählt ist aus Polyvinylpyrrolidon, Vinylpyrrolidon/Vinylacetat Copolymeren und Polyvinylcaprolactam.

8. Mittel nach einem der vorstehenden Ansprüche, **dadurch gekennzeichnet, dass** es in Form einer Lotion, eines Gels, einer Non-Aerosol Sprühlotion in Kombination mit einer mechanischen Vorrichtung zum Versprühen, in Form eines Aerosol-Haarsprays in Kombination mit einem Treibmittel, in Form eines Aerosol-Schaumes in Kombination mit einem Treibmittel oder in Form eines Non-Aerosol Schaumes in Kombination mit einer mechanischen Vorrichtung zum Verschäumen vorliegt.

9. Aerosol-Haarsprayprodukt bestehend aus einer druckfesten Verpackung, einer Zusammensetzung gemäß einem der Ansprüche 1 bis 8, welche zusammen mit einem Treibmittel in der druckfesten Verpackung abgefüllt ist und einem Sprühsystems zum Versprühen der Zusammensetzung, wobei die Zusamensetzung einen Gehalt aufweist an
(A) 1 bis 15 Gew.% eines zu 50 bis 100% neutralisierten amphoteren Copolymeren, gebildet aus Alkylacrylamid, Alkylaminoalkylmethacrylat und zwei oder mehr Monomeren bestehend aus Acrylsäure, Methacrylsäure und deren einfachen Alkylestern, wobei mindestens eines der Monomere eine Säuregruppe trägt und die Alkylgruppen 1 bis 10 C-Atome aufweisen,
(B) 0,01 bis 5 Gew.% eines zu 50 bis 100% neutralisierten anionischen Copolymeren, ausgewählt aus Polymeren, welche gebildet sind aus mindestens einer ersten Monomerart, ausgewählt aus Acryl- und Methacrylsäure und mindestens einer zweiten Monomerart, ausgewählt aus Acrylsäurealkylestern, Methacrylsäurealkylestern, Acrylamid und Methacrylamid, wobei die Alkylgruppen 1 bis 5 C-Atome aufweisen,
(C) 0,01 bis 5 Gew.% Vinylpyrrolidon/Vinylacetat Copolymer
(D) 10 bis 60 Gew.% eines C2- oder C3-Alkohols und
(E) 10 bis 85 Gew.% mindestens eines Aerosol-Treibmittels.

## Claims

1. Hair treatment composition with a content of
(A) at least one film-forming, hair-setting, amphoteric copolymer which is formed from at least one first type of monomer which has at least one neutralized or unneutralized acid group, and at least one second type of monomer which has at least one neutralized or unneutralized amine group,
(B) at least one film-forming, hair-setting anionic polymer and
(C) at least one film-forming, hair-setting nonionic polymer chosen from vinyllactam homopolymers or copolymers.

2. Hair treatment composition according to Claim 1, **characterized in that** the amphoteric polymer (A) is used in an amount of from 1 to 15% by weight, the anionic polymer (B) is used in an amount of from 0.01 to 5% by weight and the nonionic polymer (C) is used in an amount of from 0.01 to 5% by weight.

3. Hair treatment composition according to one of the preceding claims, **characterized in that** the quantitative ratio of amphoteric polymer to anionic polymer (A):(B) is from 1:1 to 200:1 and the quantitative ratio of amphoteric polymer to the nonionic polymer (A):(C) is from 1:1 to 200:1.

4. Composition according to one of the preceding claims, **characterized in that** the amphoteric polymer (A) is chosen from copolymers formed by alkylacrylamide, alkylaminoalkyl methacrylate and one, two or more monomers consisting of acrylic acid, methacrylic acid and simple alkyl esters thereof, where at least one of the monomers carries an acid group, where the alkyl groups each have 1 to 10 carbon atoms.

5. Composition according to one of the preceding claims, **characterized in that** the anionic polymer (B) is chosen from polymers or copolymers which are formed from at least one type of monomer, which is a free-radically polymerizable, ethylenically unsaturated carboxylic acid which is chosen from acrylic acid, methacrylic acid, crotonic acid, maleic acid, maleic anhydride and maleic monoesters.

6. Composition according to Claim 5, **characterized in that** the anionic polymer (B) is chosen from copolymers which are formed from at least one first type of monomers chosen from acrylic acid and methacrylic acid, and at least one second type of monomer chosen from acrylic alkyl esters, methacrylic alkyl esters, acrylamide, methacrylamide and esters of vinyl alcohol, where the alkyl groups have 1 to 5 carbon atoms.

7. Composition according to one of the preceding claims, **characterized in that** the nonionic polymer (C) is chosen from polyvinylpyrrolidone, vinyl-pyrrolidone/vinyl acetate copolymers and polyvinylcaprolactam.

8. Composition according to one of the preceding claims, **characterized in that** it is in the form of a lotion, a gel, a nonaerosol spray lotion in combination with a mechanical spraying device, in the form of an aerosol hairspray in combination with a propellant, in the form of an aerosol foam in combination with a propellant or in the form of a nonaerosol foam in combination with a mechanical foaming device.

9. Aerosol hairspray product consisting of a pressure-tight packaging, a composition according to one of Claims 1 to 8 which is bottled together with a propellant in the pressure-tight packaging, and a spray system for spraying the composition, where the composition has a content of
(A) 1 to 15% by weight of a 50 to 100%-neutralized amphoteric copolymer formed from alkylacrylamide, alkylaminoalkyl methacrylate and two or more monomers consisting of acrylic acid, methacrylic acid and simple alkyl esters thereof, where at least one of the monomers carries an acid group and the alkyl groups have 1 to 10 carbon atoms,
(B) 0.01 to 5% by weight of a 50 to 100%-neutralized anionic copolymer chosen from polymers formed from at least one first type of monomer chosen from acrylic acid and methacrylic acid, and at least one second type of monomer chosen from acrylic alkyl esters, methacrylic alkyl esters, acrylamide and methacrylamide, where the alkyl groups have 1 to 5 carbon atoms,
(C) 0.01 to 5% by weight of vinylpyrrolidone/vinyl acetate copolymer
(D) 10 to 60% by weight of a C2- or C3-alcohol and
(E) 10 to 85% by weight of at least one aerosol propellant.

## Revendications

1. Composition de traitement capillaire contenant
(A) au moins un copolymère amphotère filmogène, fixant les cheveux, qui est formé à partir d'au moins un premier type de monomère comportant au moins un groupe acide neutralisé ou non neutralisé et d'au moins un second type de monomère comportant au moins un groupe amino neutralisé ou non neutralisé,
(B) au moins un polymère anionique filmogène, fixant les cheveux et
(c) au moins un polymère non ionique filmogène, fixant les cheveux, choisi parmi les homo- ou copolymères de vinyl-lactame.

2. Composition de traitement capillaire selon la revendication 1, **caractérisée en ce que** le polymère amphotère (A) est utilisé en une quantité de 1 à 15 % en poids, le polymère anionique (B) est utilisé en une quantité de 0,01 à 5 % en poids et le polymère non ionique (C) est utilisé en une quantité de 0,01 à 5 % en poids.

3. Composition de traitement capillaire selon l'une quelconque dés revendications précédentes, **caractérisée en ce que** le rapport de la quantité du polymère amphotère à celle du polymère anionique (A):(B) va de 1:1 à 200:1 et le rapport de la quantité du polymère amphotère à celle du polymère non ionique (A):(C) va de 1:1 à 200:1.

4. Composition selon l'une quelconque des revendications précédentes, **caractérisée en ce que** le polymère amphotère (A) est choisi parmi des copolymères formés à partir d'alkylacrylamide, de méthacrylate d'alkylaminoalkyle et d'un, de deux ou de plus de deux monomères constitués d'acide acrylique, d'acide méthacrylique et de leurs monoesters alkyliques, au moins l'un des monomères portant un groupe acide, les groupes alkyle comportant chacun de 1 à 10 atomes de carbone.

5. Composition selon l'une quelconque des revendications précédentes, **caractérisée en ce que** le polymère anionique (B) est choisi parmi des polymères ou copolymères qui sont formés à partir d'au moins un type de monomère consistant en un acide carboxylique à insaturation éthylénique, susceptible de polymérisation radicalaire, qui est choisi parmi l'acide acrylique, l'acide méthacrylique, l'acide crotonique, l'acide maléique, l'anhydride maléique et les monoesters d'acide maléique.

6. Composition selon la revendication 5, **caractérisée en ce que** le polymère anionique (B) est choisi parmi des copolymères qui sont formés à partir d'au moins un premier type de monomère, choisi parmi l'acide acrylique et l'acide méthacrylique, et d'au moins un second type de monomère, choisi parmi des acrylates d'alkyle, des méthacrylates d'alkyle, l'acrylamide, le méthacrylamide et des esters d'alcool vinylique, les groupes alkyle comportant de 1 à 5 atomes de carbone.

7. Composition selon l'une quelconque des revendications précédentes, **caractérisée en ce que** le polymère non ionique (C) est choisi parmi la polyvinylpyrrolidone, des copolymères vinylpyrrolidone/acétate de vinyle et le polyvinylcaprolactame.

8. Composition selon l'une quelconque des revendications précédentes, **caractérisée en ce qu'**elle se trouve sous forme d'une lotion, d'un gel, d'une lotion à pulvériser non-aérosol, en association avec un dispositif mécanique pour la pulvérisation, sous forme d'une laque à pulvériser en aérosol en association avec un propulseur, sous forme d'une mousse pour aérosol en association avec un propulseur, ou sous forme d'une mousse non-aérosol en association avec un dispositif mécanique pour le moussage.

9. Produit de type laque à pulvériser en aérosol, constitué d'un emballage tenant la pression, d'une composition selon l'une quelconque des revendications 1 à 8, qui est introduite conjointement avec un propulseur dans l'emballage tenant la pression et d'un système de pulvérisation pour la pulvérisation de la composition, la composition contenant
(A) 1 à 15 % en poids d'un copolymère amphotère neutralisé à raison de 50 à 100 %, formé à partir d'alkylacrylamide, de méthacrylate d'alkylaminoalkyle et de deux ou de plus de deux monomères constitués d'acide acrylique, d'acide méthacrylique et de leurs monoesters alkyliques, au moins l'un des monomères portant un groupe acide et les groupes alkyle comportant de 1 à 10 atomes de carbone,
(B) 0,01 à 5 % en poids d'un copolymère anionique neutralisé à raison de 50 à 100 %, choisi parmi des polymères qui sont formés à partir d'au moins un premier type de monomère, choisi parmi l'acide acrylique et l'acide méthacrylique, et d'au moins un second type de monomère, choisi parmi des acrylates d'alkyle, des méthacrylates d'alkyle, l'acrylamide et le méthacrylamide, les groupes alkyle comportant de 1 à 5 atomes de carbone,
(C) 0,01 à 5 % en poids d'un copolymère vinylpyrrolidone/acétate de vinyle,
(D) 10 à 60 % en poids d'un alcool en C₂ ou C₃ et
(E) 10 à 85 % en poids d'au moins un propulseur pour aérosol.
